# EUROPEAN PATENT APPLICATION

(11) **EP 4 851 614 A1**
(43) Date of publication of application: **09.12.2026**
(21) Application number: 26177512.6
(22) Date of filing: 08.05.2026
(51) Int. Cl.: G01N 27/08, G01N 33/18, G01N 31/00

(54) **SYSTEMS AND METHODS FOR MEASURING CONCENTRATIONS OF ONE OR MORE IMPURITIES IN WATER**

(30) Priority: 05.06.2025 US 202519229506
(71) Applicant: Mettler-Toledo Thornton, Inc., Billerica MA 01821 (US)
(72) Inventor: Kokane, Sanjay, 422601 Akole (IN); Bevilacqua, Anthony, Medford, MA 02155 (US); Gayner, Chhatrasal, 441106 Nagpur (IN); Kesarkar, Sagar, Didcot, OX11 7AZ (GB); Reijnders, Anjan, Billerica, 01821 (US)
(74) Representative: Mettler-Toledo

(57) **Abstract**

Devices, systems, and methods for detecting concentrations of one or more impurities in water are disclosed. A measurement subsystem for a flow of analyte includes a first and second conductivity sensor with an interposed electrochemical module. Measurement data from the measurement subsystem, including conductivity measurements from the first and second conductivity sensors, is received at an analysis subsystem. A difference between the conductivity measurements is determined. The difference is analyzed to determine an estimated concentration of each of the one or more of the impurities.

## Description

### Technical Field

Exemplary embodiments relate generally to apparatuses, systems, and methods for measuring concentrations of one or more impurities in water.

### Background and Summary of the invention

Various devices are known for analyzing fluids, such as to determine concentration levels of various elements of the fluid. Such devices may be important for measuring purity of water, for example, and may be particularly useful for sensitive applications, such as pharmaceutical applications and/or semiconductor manufacturing, by way of non-limiting example. For example, a maximum level of concentration of various compounds may be required to meet various regulatory or scientific standards (*e.g*., requirements) and/or otherwise ensure quality.

Halides are an impurity naturally present in ground water. In water purification treatments, halides are sometimes removed by reverse osmosis, electro-deionization and/or resin columns. Typically, after multiple purification steps, halide concentrations can be lowered down to near-zero parts per billion (ppb) (or other system of units measurement), such as to meet certain ultra-pure water standards, for example. In other examples, without limitation, halides can be formed from photodegradation of halogenated organics.

The concentration of halides can be important to control. In power plants, for example, chloride contamination of steam can contribute to stress corrosion cracking, corrosion fatigue, and/or pitting of steam turbines. Halides are highly corrosive in nature when present in the water/steam cycle of a power plant. They can damage critical expensive components of a power plant such as turbines, boilers, and steam generator tubes, which can lead to unplanned shutdowns and reduced power generation efficiency. Different chloride (halide) detection technologies include electrochemical method(s), potentiometric titration method(s), spectrophotometric method(s), chromatography method(s), and volumetric silver nitrate method(s) with variable detection limits. However, existing technologies fall short in several aspects, such as by requiring multiple manual steps, high operational cost, laboratory-based instrumentation, combinations thereof, and the like. In at least one aspect, existing technologies are unable to provide ongoing, online, and/or continuous measurements in ultra-pure water applications.

For example, current electrochemistry approaches are limited in the ability to measure a linear response of current or potential due to the low ionic strength in pure water which may provide a detection limit of the analyte to about 100 µg/L (ppb). One of the challenges in electrochemistry is to measure the linear response of an analyte in pure water without adding any electrolyte. The addition of electrolyte provides the ionic strength required to measure a linear response of current or potential with analyte concentrations. The addition of electrolyte (reagents like formic acid) makes the system more complex, particularly for continuous measurements given the time needed. Other known methods, such as spectrophotometric, potentiometric, and chromatography are not suitable for ongoing measurements, are reagent dependent, and are complex.

Total organic carbon (TOC) concentration can be another important metric to control. Conventional TOC measurement systems are commonly designed to utilize a direct conductometric method and they generally involve three main steps. First, the concentration of inorganic carbon is determined based on a first conductivity measurement made before the oxidation chamber. Second, organics present in the analyte fluid are oxidized to CO₂ and its concomitant ions, HCO₃⁻ and H⁺. Third, a second conductivity measurement is made after the oxidation chamber, resulting in an increase in conductivity due to the newly formed ions. The concentration of TOC is determined based on a difference between the second conductivity measurement and the first conductivity measurement. Direct conductometric detection method is not an ion-selective method and measures all the conductive ions in the water along with newly formed HCO₃⁻ and H⁺ (from the generated CO₂). Such conventional TOC measurement systems can therefore measure false high TOC recoveries for halogenated organic compounds like trihalomethanes (THMs). The term recovery, or variations thereof for grammatical/clarity purposes, refers to an amount of material measured or reported, divided by an actual amount present. The high TOC recovery of chlorinated organic compounds with the direct conductometric sensor is due, at least in part, to the formation of HCl in the oxidation reactor, thereby resulting higher conductivity than from ions resulting from CO₂ alone. For example, if the chloroform is present in the water, it forms HCl along with CO₂ in the oxidation chamber. The conductivity of HCl is very high as compared to CO₂ and hence these conventional approaches tend to over report the TOC concentration. These disclosures provide THM-compensated TOC measurements by measuring the THM concentration in the analyte fluid followed by removing the THMs induced conductivity before the TOC calculation.

One aspect of the present invention relates to a system for measuring estimated concentrations of one or more impurities in water according to claim 1.

Another aspect of the present invention relates to a method for measuring estimated concentrations of one or more impurities in water according to claim 14.

A further aspect of the present invention relates to a system for measuring estimated concentrations of one or more impurities in water according to claim 15.

Preferred embodiments of the aspects of the present invention are stated in the corresponding dependent claims and are described below.

The inventive devices, systems, and methods disclosed herewith provide for measuring concentrations of one or more impurities in water, such as on an ongoing basis and/or in at least substantially real time. In exemplary embodiments, the concentration of halides in water is determined using a combination of electrochemical techniques and differential conductivity measurements. By including ultraviolet exposure upstream, the concentration of THMs may be determined. By adding an additional conductivity measurement upstream of the UV exposure, TOC concentration may be determined. In this way, an interference compensated TOC measurement may be determined, such as from the TOC concentration compensated for the concentration of halides and/or THMs, which may each be separately reported.

A measurement subsystem for analyzing a flow of analyte (e.g., water) includes an electrochemical module interposed between a first and second conductivity sensor. An analysis subsystem receives data from the measurement subsystem, including measurements from the first and second conductivity sensors to determine a difference between a measurement from the first conductivity sensor upstream of the electrochemical module and a measurement from the second conductivity sensor downstream of the electrochemical module. This difference may be used to determine an estimated concentration of halides in the analyte.

The electrochemical module may include one or more anodes and one or more cathodes, preferably arranged in an alternating fashion with inert material interposed. The anode(s) and/or cathode(s) may include pores that allow the analyte fluid (e.g., water) to pass through the electrochemical module. The electrochemical module may take other forms. For example, without limitation, the electrochemical module may include one or more sheets providing anode(s) and/or cathode(s), preferably arranged into alternating layers with sheets of inert material interposed. The analyte fluid may thereby pass along and between the layers.

In other exemplary embodiments, without limitation, an ultraviolet (UV) light source may be provided upstream of the first conductivity sensor. This may allow for measurement of THM concentration, such as one of the one or more impurities.

In other exemplary embodiments, without limitation, a third conductivity sensor may be provided upstream of the UV light source. The difference between the conductivity measurements from the third and first conductivity sensors may provide an estimate of total organic carbon (TOC) concentration, which may be one of the one or more impurities. This difference may be analyzed with the difference between the conductivity measurements from the first and second conductivity sensors to determine an interference compensated TOC concentration measurement, such as by compensating for the determined presence of halides and/or THMs. This may allow for measurement of TOC and/or interference free TOC, which may be one of the one or more impurities.

The conductivity differences may be scaled, multiplied by various coefficients or other factors, and/or analyzed by way of an algorithm to arrive at the one or more impurity measurements. For example, without limitation, the coefficients may be derived from a regression analysis of conductivity-concentration curve of each of the one or more impurities and/or a three-dimensional error analysis mesh. The resulting concentration measurement(s) may be locally and/or remotely output, such as in various graphical formats, stored for historical or other analysis, combinations thereof, or the like.

As used herein, the term "water", or variations thereof for grammar and/or clarity, refers to commonly supplied water, preferably at least meeting generally accepted potability standards, such as from a utility water supply, ground water source, surface source, internal supply, storage, combinations thereof, or the like, which may contain one or more impurities and/or additives and/or may be at least partially purified (e.g., filtered and/or treated). The term "analyte fluid", or variations thereof for grammar and/or clarity, refers to portions of water being analyzed.

Provided hereinafter is a listing of certain non-limiting examples of embodiments of the inventive technology.

In an exemplary embodiment, a system for measuring estimated concentrations of one or more impurities in water includes a measurement subsystem for a flow of analyte. The measurement subsystem includes a first conductivity sensor, an electrochemical module positioned downstream of the first conductivity sensor, and a second conductivity sensor positioned downstream of the electrochemical module. An analysis subsystem is in electronic communication with the measurement subsystem and includes one or more non-transitory electronic storage devices with software instructions, which when executed, configure one or more processors to: receive measurement data from the measurement subsystem for the flow of the analyte, said measurement data comprising conductivity measurements from the first conductivity sensor and the second conductivity sensor, determine a difference between a conductivity measurement from the second conductivity sensor and an associated conductivity measurement from the first conductivity sensor, and analyze the difference to determine an estimated concentration of each of the one or more of the impurities. This may allow for measurement of concentrations of one or more impurities in water, such as on an ongoing basis and/or in at least substantially real time.

In an exemplary embodiment, the one or more non-transitory electronic storage devices of the analysis subsystem may include additional software instructions, which when executed, configure the one or more processors to: time correlate the conductivity measurements from the second conductivity sensor with the associated conductivity measurements from the first conductivity sensor. This may allow for the same quantity of analyte to be measured.

In an exemplary embodiment, the analysis subsystem is configured to output the estimated concentration of halide, such as for a user to view.

In an exemplary embodiment, the electrochemical module includes one or more anodes, each comprising silver, one or more cathodes, arranged in an alternating fashion with the one or more anodes, and inert material interposed between each of the one or more anodes and adjacent one of the one or more cathodes. For example, the one or more of cathodes includes one or more of: stainless steel, platinum, titanium, silver, gold, graphite, and carbon. For example, the inert material comprises at least one of: polyvinylidene fluoride, polyether ether ketone, perfluoroalkoxy alkane, and polypropylene. In an exemplary embodiment, each of the one or more anodes and each of the one or more cathodes include a plurality of pores for the flow of the analyte to travel through. In another exemplary embodiment, each of the one or more anodes and each of the one or more cathodes are provided in the form of sheets which are stacked in an alternating fashion with the inert material interposed therebetween to allow the flow of the analyte to travel therebetween. Some or all of the foregoing may provide effective analysis of impurities, such as halides.

In an exemplary embodiment, the measurement subsystem comprises one or more oxidation devices, such as ultraviolet light source(s), such as which generate photons shorter than 200 nm wavelength, and said one or more oxidation devices is positioned upstream of the first conductivity sensor. This may facilitate the measurement of the estimated concentration of trihalomethane.

In an exemplary embodiment, the measurement subsystem includes a third conductivity sensor located upstream of the one or more oxidation devices, the measurement data comprises conductivity measurements from the third conductivity sensor, and the one or more non-transitory electronic storage devices of the analysis subsystem comprise additional software instructions, which when executed, configure the one or more processors to: determine a difference between the conductivity measurement from the first conductivity sensor and an associated conductivity measurement from the third conductivity sensor ("second difference") to determine an estimated concentration of total organic carbon; and analyze the second difference with the difference to determine an interference compensated estimated concentration of total organic carbon. This allows the measurement of an interference compensated estimated concentration of total organic carbon.

In an exemplary embodiment, the one or more non-transitory electronic storage devices of the analysis subsystem comprise additional software instructions, which when executed, configure the one or more processors to: receive the measurement data, determine the difference, and analyze the difference in substantially real time on an ongoing basis such that the estimated concentration of each of the one or more of the impurities is determined over time. The analysis subsystem may be configured to transmit the estimated concentration of each of the one or more of the impurities to at least one remote electronic device for display. The foregoing may allow for real time, or substantially real time, estimated concentration readings.

In an exemplary embodiment, the components of the measurement subsystem are in direct fluid connection with one another and the analysis subsystem is remote from the measurement subsystem. This may allow for remote monitoring.

In an exemplary embodiment, the analysis subsystem may be configured to analyze the difference to determine the estimated concentration of each of the one or more of the impurities at least in part by multiplying the difference by one or more coefficients. The one or more coefficients may include a plurality of coefficients, at least one of which is derived by the regression analysis of the conductivity-concentration curve of a respective one of the one or more impurities. The foregoing may allow for more accurate estimated concentration determination.

Various of the foregoing embodiments may be carried out in combination and/or as part of a method.

Further features and advantages of the systems and methods disclosed herein, as well as the structure and operation of various aspects of the present disclosure, are described in detail below with reference to the accompanying figures.

### Brief description of drawings

In addition to the features mentioned above, other aspects of the present invention will be readily apparent from the following descriptions of the drawings and exemplary embodiments, wherein like reference numerals across the several views refer to identical, similar, or equivalent features, and wherein:
- Fig. 1: is a plan view of an exemplary schematic of a system for measuring concentration of one or more impurities in water;
- Fig. 2: is a detailed, sectional view of an exemplary electrochemical module for the system of figure 1;
- Fig. 3: is a graphical representation of exemplary conductivity measurements over time at various dosing for the electrochemical module of figure 2;
- Fig. 4a: is a detailed, sectional view of another exemplary electrochemical module for the system of figure 1;
- Fig. 4b: is a top, sectional view of the electrochemical module of figure 4A, taken along section line A-A thereof;
- Fig. 4c: is a detailed, sectional view of another exemplary electrochemical module for the system of figure 1;
- Fig. 4d: is a top, sectional view of the electrochemical module of figure 4A, taken along section line A-A thereof;
- Fig. 5: is a perspective view of layers of the electrochemical module of figure 4 illustrated in isolation;
- Fig. 6: is a plan view of another exemplary embodiment of the system for measuring concentration of one or more impurities in water;
- Fig. 7: is a plan view of another exemplary embodiment of the system for measuring concentration of one or more impurities in water;
- Fig. 8: is a graphical representation of exemplary responses of the electrochemical module of figure 2 under control dosing of different concentration of THMs (CHCl₃) at the system of figure 7;
- Fig. 9: is a graphical representation of another collection of exemplary responses of the system of figure 2 under control dosing of different concentration of CHBrCl₂ at the system of figure 7; and
- Fig. 10: is a front, sectional view of another exemplary embodiment of the system for measuring concentration of one or more impurities in water.

### Detailed Description of exemplary embodiments

Various embodiments of the present invention will now be described in detail with reference to the accompanying drawings. In the following description, specific details such as detailed configuration and components are merely provided to assist the overall understanding of these embodiments of the present invention. Therefore, it should be apparent to those skilled in the art that various changes and modifications of the embodiments described herein can be made without departing from the scope and spirit of the present invention. In addition, descriptions of well-known functions and constructions are omitted for clarity and conciseness.

Embodiments of the invention are described herein with reference to illustrations of idealized embodiments (and intermediate structures) of the invention. As such, variations from the shapes of the illustrations as a result, for example, of manufacturing techniques and/or tolerances, are to be expected. Thus, embodiments of the invention should not be construed as limited to the particular shapes of regions illustrated herein but are to include deviations in shapes that result, for example, from manufacturing.

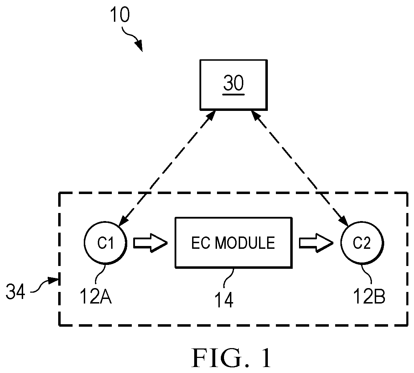

**FIGURE 1** illustrates an exemplary system 10 for measuring concentrations of one or more impurities in water. The system 10 may include a first conductivity sensor 12A located upstream of an electrochemical module 14 and a second conductivity sensor 12B located downstream of the electrochemical module 14. At least the first and second conductivity sensors 12A, 12B and the electrochemical module 14 may define and/or comprise at least part of a measurement subsystem 34. Optionally, the measurement subsystem 34 may include, by way of non-limiting example, various related components (e.g., power supplies, controllers, fluid passageway(s), combinations thereof, or the like).

At least the first and second conductivity sensors 12A, 12B may be in electronic communication with an analysis subsystem 30, such as in a wired and/or wireless fashion. Data from the conductivity sensors 12A, 12B, such as indicating conductivity measurements, may be communicated to the analysis subsystem 30. The analysis subsystem 30 may comprise one or more non-transitory electronic storage devices and/or one or more processors. The analysis subsystem 30 may be part of the measurement subsystem 34 and/or separate therefrom.

Optionally, the electrochemical module 14 may be in electronic communication with the analysis subsystem 30, which may exert operational control over, provide operational power to, and/or receive data (e.g., indicating operational status) from the same, though such is not required.

The sensors 12A, 12B and electrochemical module 14 may be provided along one or more fluid passageways, such as for the analyte fluid (e.g., water). The fluid passageways may include pipes, tubes, tanks, combinations thereof, or the like. The sensor(s) 12A, 12B and electrochemical module (EC Module) 14 may be provided along a same or different one of the fluid passageway(s). The fluid passageway(s) may take on various forms and/or shapes. Valves and other components may be interposed.

The analysis subsystem 30 may be configured to receive conductivity data from the sensors 12A, 12B. The analyte flow (e.g., water stream) containing halides may pass through the electrochemical module 14. The conductivity of the analyte fluid is measured at the first conductivity sensor 12A which is placed upstream of the electrochemical module 14 - this measurement sometimes referred to as "C1" herein. In exemplary embodiments, without limitation, the analyte fluid may be contacted with silver electrodes. The anodic potential is provided to the silver electrodes while maintaining close contact of silver electrodes with the analyte fluid containing halide ions. The conductivity of the analyte fluid is measured at the second conductivity sensor 12B placed downstream of the electrochemical module 14 - this measurement sometimes referred to as "C2" herein. The conductivity of the analyte fluid before the electrochemical module 14, i.e., C1, and after the electrochemical module, i.e., C2, are measured to determine the halide concentrations. For example, without limitation, the analysis subsystem 30 may be configured to determine the difference in conductivities (e.g., C2-C1) to determine the halide concentration in the analyte fluid. The analysis subsystem 30 may be configured to determine a difference between the conductivity measurements (e.g., C2 - C1) to determine an estimated concentration of halides within the analyzed fluid, which may be at least one of the one or more measured impurities.

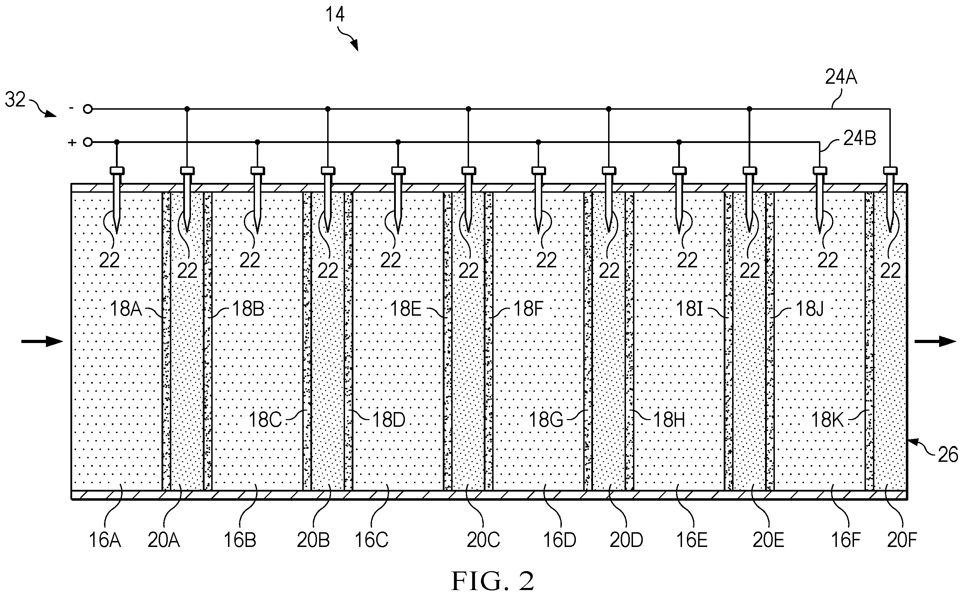

**FIGURE 2** illustrates an exemplary embodiment of the electrochemical module 14. The electrochemical module 14 may include one or more anodes 16A-16F. The electrochemical module 14 may include one or more cathodes 20A-20F. The number, size, shape, and/or arrangement of cathodes 20 is exemplary and not intended to be limiting. The number, size, shape, and/or arrangement of the anodes 16, cathodes 20, and/or portions of inert material 18A-18K is exemplary and not intended to be limiting. In exemplary embodiments, without limitation, multiple anodes 16A-16F and a corresponding number of cathodes 20A-20F are provided in series in an alternating fashion within a housing 26, such as with one of the portions of inert material 18A-18K interposed between each. In exemplary embodiments, without limitation, each of the anodes 16 and each of the cathodes are of a thickness between 2-6mm and may be the same or different, though other thicknesses may be utilized in other embodiments. As used herein, the term "inert", or variations thereof for grammar and/or clarity, such as in the "inert material 18A-18K", refers to a material which acts as an electrical insulator (e.g., non-conductive or minimally conductive, such as less than 10-8 S/m by way of non-limiting example) and is chemically non-reactive, or minimally reactive, at least to the anodes 16A-16F, the cathodes 20A-20F and any chemical substances in the analyte stream.

Connectors 22 may extend into each of the anodes 16 and cathodes 20. A first set of one or more wires 24B may extend to each of the connectors 22 electrically connected to each of the anodes 16. A second set of one or more wires 24A may extend to each of the connectors 22 electrically connected to each for the cathodes 20. The wires 24A, 24B may be connected to one or more power sources 32. The power sources may optionally include the analysis subsystem 30.

At least the anode(s) 16, cathode(s) 20, and portion(s) of inert material 18 may be located within the housing 26. The connectors 22 may optionally extend outside the housing 26 so that the wires 24 may be attached. The power source(s) 32 may be connected to the wires 24, such as at a location outside of the housing 26, though other arrangements may be utilized. The housing 26 may be generally tubular in shape, and the anode(s) 16, cathode(s) 20 and/or inert material portion(s) 18 may be generally cylindrical in shape to fit therein, though other shapes and/or arrangements may be utilized.

Some or all of the anode(s) 16, cathode(s) 20, and/or portion(s) of inert material 18 may comprise one or more pores, such as to allow the analyte to flow therethrough. For example, without limitation, some or all of the anode(s) 16, cathode(s) 20, and/or portion(s) of inert material 18 may be provided as filaments (e.g., in spun and/or wool type form), wire, fibers, porous mesh, porous sheets, combinations thereof or the like, to provide the pores. In exemplary embodiments, without limitation, the inert material 18 is provided as a series of membranes, such as with pore size >0.45µm and/or thickness 125µm.

In exemplary embodiments, without limitation, electrochemical conversion of chloride to silver chloride may require the standard reduction potential of +0.2223 V vs Ag/AgCl, while bromide to silver bromide requires 0.071 V vs Ag/AgCl, and iodide to silver iodide may require -0.152 V vs Ag/AgCl (Eq. 1, Eq. 2, Eq. 3). This reflects the tendency of formation of silver halides follows I > Br > Cl. The oxidation-reduction potentials of silver with halides are given below: ${AgI}_{\left(s\right)} + {e}^{-} ↔ {Ag}_{\left(s\right)} + {I}_{aq .}^{-} - 0.152 V vs SHE$ Equation 1 ${AgBr}_{\left(s\right)} + {e}^{-} ↔ {Ag}_{\left(s\right)} + {Br}_{aq .}^{-} + 0.071 V vs SHE$ Equation 2 ${AgCl}_{\left(s\right)} + {e}^{-} ↔ {Ag}_{\left(s\right)} + {Cl}_{aq .}^{-} + 0.2223 V vs SHE$ Equation 3 ${Ag}_{aq .}^{+} + {e}^{-} ↔ {Ag}_{\left(s\right)} + 0.7996 V vs SHE$ Equation 4

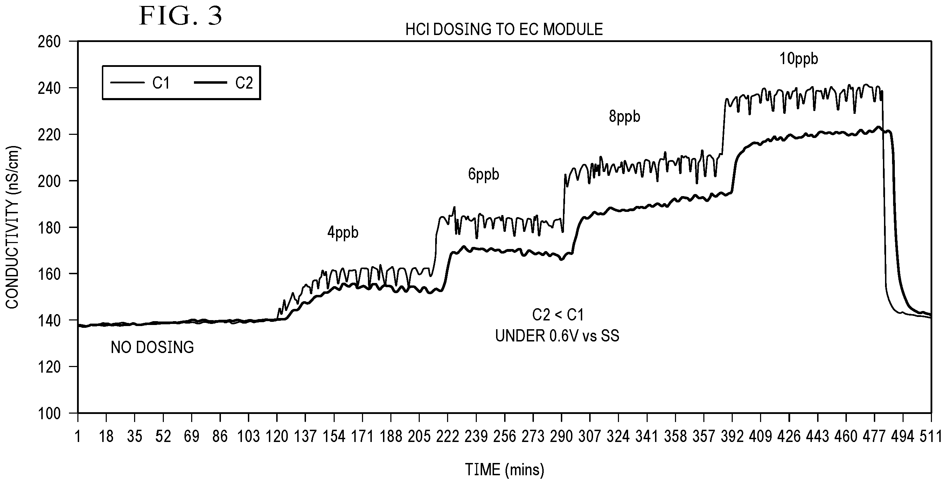

The silver oxidation potential of +0.7996V vs Standard Hydrogen Electrode (SHE) may limit the application of potential to the module. Low oxidation-reduction potential of above electrochemical reaction may provide selectivity of halide detection on the silver electrode. In a first step, after applying anodic potential to the Ag electrode, the Ag electrode may become polarized. In the presence of halides in the analyte fluid, halides may be adsorbed/attracted towards the silver surface. Under sufficient potential, a partially oxidized Ag electrode may convert the halide ions to silver halide on the silver electrode surface; (AgX, where X=Cl, Br, I). For example, without limitation, chloride ions may get electrochemically deposited on the silver surface under 0.2223V vs Ag/AgCl. Conversion of halides to insoluble silver halide may result in loss of halide conductivity in the analyte fluid. Under sufficient potential, all halides present in the analyte fluid may get chemisorbed at the silver electrode surface, thereby providing direct proportionality between conductivity difference measured before and after the electrochemical module 14, and thereby establishing the halide concentration. This may provide ongoing (e.g., continuous) measurements of the halide concentration in purified water without any addition of chemical reagent, such as to provide at least substantially real-time monitoring of halide contamination in purified water. As demonstrated by **FIGURE 3****,** different concentrations of HCl dosing are provided to the electrochemical module 14 having silver anodes 16 under continuous potential.

The analysis subsystem 30 may utilize a chemical-based algorithm which utilizes the differential conductivity measurement, e.g., C2-C1, to determine the concentration of halides present in the analyte fluid. In exemplary embodiments, the algorithm includes multiplying the differential conductivity measurement by at least one compensation factor. The compensation factor(s) may include one or more efficiency corrections and/or polynomial coefficients, which may be derived from a regression analysis of samples with known quantities of certain halides. In exemplary embodiments, without limitation, the following equation is utilized, such as along with further compensations, conversions, scaling, combinations thereof, or the like: $Halide concentration = A 0 + \left(A1∗Δσ\right) + \left(A2∗Δ{σ}^{2}\right)$ Equation 5 Where A0, A1, and A2 are coefficients derived from the regression analysis and Δ*σ* is the differential conductivity measurement (e.g., C2 - C1).

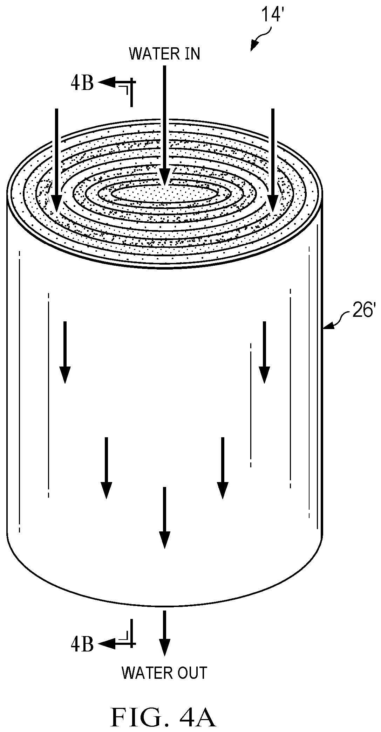

**FIGURE 4A** through **FIGURE 4D** illustrate other exemplary embodiments of the electrochemical module 14'. The same or similar items may be numbered similarly but with the addition of a prime (') (e.g., 14 to 14'). The anode(s) 16A', 16B' cathode(s) 20A', 20B' and/or portion(s) of inert material 18A', 18B' may be provided as sheets, such as stacked into layers. This may allow the interfering ions to remain relatively close to the electrode surface while passing through the electrochemical module 14'. The layers are preferably arranged into a cylindrical shape such that the layers are located inside one another, though other shapes may be utilized. The housing 26' may be cylindrical in shape to support the same, though other shapes may be utilized. In this way, the analyte fluid may be provided between some or all of the layers and/or interior surface of the housing 26'.

The electrochemical module 14' may optionally have a central entrance and exit for the analyte fluid, which may be distributed between the various layers of the anodes 16', cathodes 20', and/or layers of inert material 18', such as by arrangement of the anodes 16', cathodes 20', and/or layers of inert material 18' relative to the housing 26'. Alternatively, or additionally, multiple entrances and/or exits to the housing 26' may be provided, such as between the layers of the anodes 16', cathodes 20', and/or layers of inert material 18' to distribute the analyte fluid within the electrochemical module 14'.

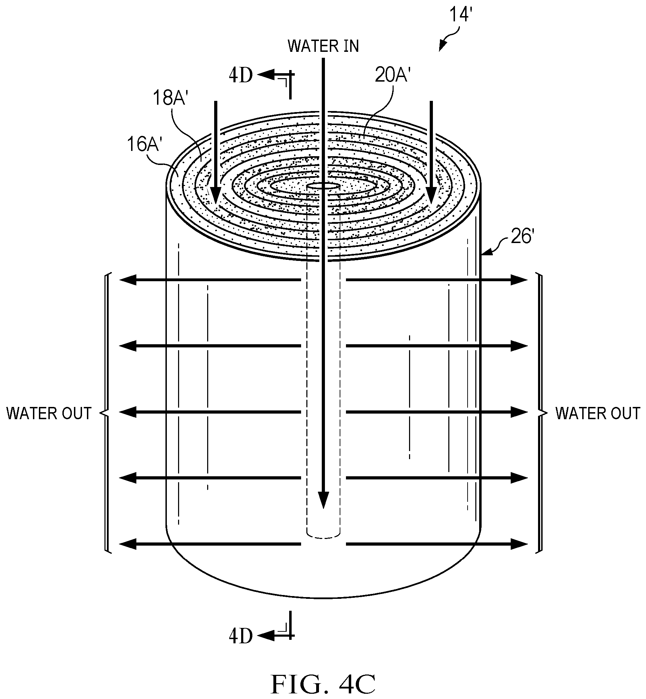

As illustrated with particular regard to figures 4A-4B, analyte may essentially pass between the layers in the electrochemical module 14' (e.g., anode(s) 16A', 16B' cathode(s) 20A', 20B' and/or portion(s) of inert material 18A', 18B'). As illustrated with particular regard to figures 4C-4D, the analyte may instead pass between the layers in the electrochemical module 14' (e.g., anode(s) 16A', 16B' cathode(s) 20A', 20B' and/or portion(s) of inert material 18A', 18B') which may be sufficiently porous or otherwise configured to accommodate the same.

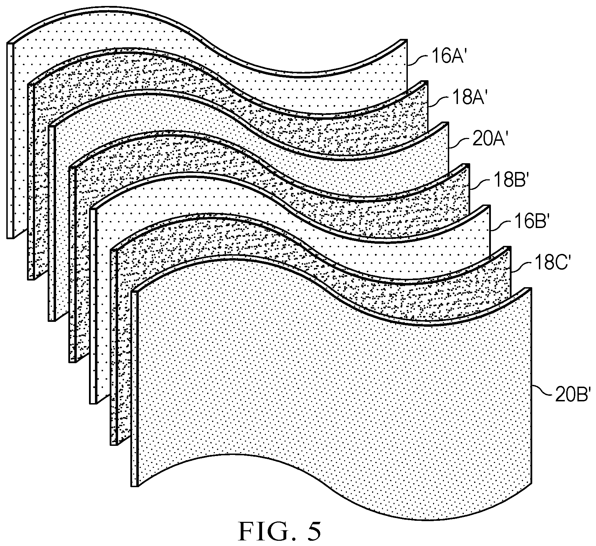

Some of the layers are illustrated outside of the housing 26' in an unfurled fashion in **FIGURE 5****.** The number, size, shape, and/or arrangement of the anodes 16', cathodes 20', and/or layers of inert material 18' is exemplary and not intended to be limiting. In exemplary embodiments, without limitation, multiple anodes 16' and a corresponding number of cathodes 20' are provided in an alternating, with one of the portions of inert material 18' interposed between each.

The anodes 16, 16' (e.g., 16A-16F, 16A'-16B') may comprise sliver, in exemplary embodiments, without limitation. The cathodes 20, 20' may comprise one or more of: stainless steel, platinum, titanium, silver, gold, graphite, and carbon, in exemplary embodiments, without limitation, and preferably stainless steel, and more preferably 316L stainless steel. The cathodes 20, 20' (e.g., comprising stainless steel) may be used as the pseudo or quasi-reference electrode and 0.6 V anodic potential may be applied to the electrodes (e.g., comprising silver) against the quasi-reference electrodes. Other materials may be utilized, such as but not limited to, gold, silver, platinum, titanium, graphite, rhodium, combinations thereof, or the like, which may have limited to no leaching and/or false conductivity.

In exemplary embodiments, without limitation, the electrochemical module 14 is configured to provide and/or allow a flow rate of at or about 8 mL/min. However, other flow rates with different geometries may be utilized.

The inert material 18, 18' comprise one or more of polytetrafluoroethylene (PTFE), polyvinylidene fluoride (PVDF), polyether ether ketone (PEEK), perfluoroalkoxy alkane (PFA), polypropylene (PP), high density polyethylene (HDPE), nylon, combinations thereof, or the like, which may provide relatively high chemical resistance and stability for example, without limitation.

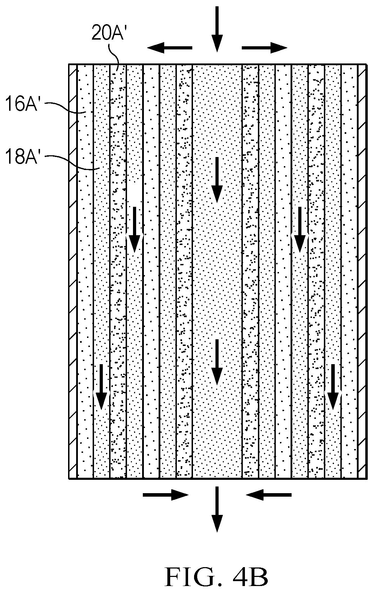

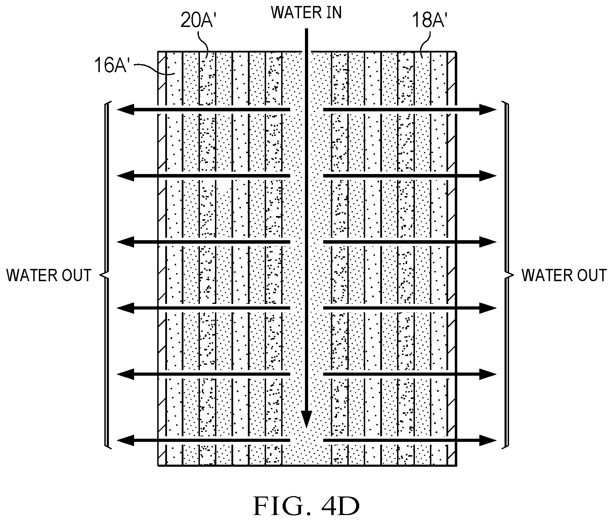

Preferably, in the embodiment of figure 2 the inert material 18 comprises porous membrane, and in the embodiment of figures 4-5, the inert material 18' comprises PVDF, though other materials may be utilized. The housing 26, 26' may comprise a same or different, preferably inert, material, such as but not limited to polytetrafluoroethylene (PTFE).

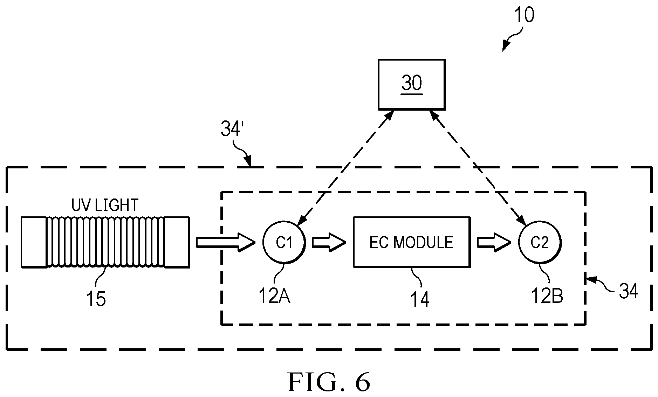

**FIGURE 6** illustrates another exemplary embodiment of the system 10, which may include the measurement subsystem 34 of figure 1 and/or one or more components thereof. One or more ultraviolet (UV) light sources 15 may be provided upstream of the first conductivity sensor 12A. At least the first and second conductivity sensors 12A, 12B, the electrochemical module 14, and the UV light source(s) 15 may define and/or comprise at least part of another exemplary embodiment of the measurement subsystem 34'. The analysis subsystem 30 may be part of, or separate from, the measurement subsystem 34'. Stated another way, the measurement subsystem 34' may include the measurement subsystem 34 of figure 1 along with the UV light source(s) 15, and optionally any related components (e.g., power supplies, controllers, fluid passageway(s), combinations thereof, or the like) for the same.

Preferably, the UV light source(s) 15 are configured to generate, when activated, photons shorter than 200 nm wavelength, which may be capable of breaking down organic compounds along with THMs, i.e., CHCl3, to halide acids and CO2.

While UV light source(s) 15 are sometimes shown and/or described, other device(s) for causing oxidation of elements in the flow of analyte may be utilized. These may include, for example and without limitation, heating elements, chemical reservoirs and/or dispensing devices, electrical connections and/or power sources, combinations thereof, or the like. The UV light source(s) 15 and/or other device(s) may be more generally referred to herein as "oxidation device(s)" or similar. Notably, these other oxidation device(s) may be used in addition to, or in substitution for, the UV light source(s) 15, even if not expressly called out as such herein.

The analysis subsystem 30 may be configured to receive conductivity data from the sensors 12A, 12B. The exposure of the analyte fluid to the UV light may cause changes to the analyte such that the analysis subsystem 30 is configured to determine an estimated concentration of trihalomethane (THM) within the analyzed fluid, which may be at least one of the one or more measured impurities.

Initially, photons from the UV light may impinge on the flowing analyte fluid to convert the THMs to halide acids, i.e., HCl, and CO2. The halide acids may immediately dissociate to hydrogen ions and halide ions. The conductivity of the analyte fluid may be measured at the first conductivity sensor 12A. Halides present in the analyte fluid directly chemisorbed on to the silver electrode surface under anodic potential may result in the loss of halide conductivity in the water. The conductivity of the analyte fluid may be measured again at the second conductivity sensor 12B.

The analysis subsystem 30 may utilize a chemical-based algorithm which utilizes the differential conductivity, e.g., C2-C1 (with UV light exposure), to determine the concentration of THMs present in analyte fluid. In exemplary embodiments, the algorithm includes multiplying the differential conductivity measurement by at least one compensation factor. The compensation factor(s) may include one or more efficiency corrections and/or polynomial coefficients, which may be derived from a regression analysis of samples with known quantities of certain halides. In exemplary embodiments, without limitation, the following equation is utilized, such as along with further compensations, conversions, scaling, combinations thereof, or the like: $Total TMHM concentration = B 0 + \left(B1∗Δσ′\right) + \left(B2∗Δ{σ}^{′ 2}\right)$ Equation 6 Where B0, B1, and B2 are coefficients derived from the regression analysis and Δ*σ*' is a compensated value of the differential conductivity measurement (e.g., C2 - C1, with UV light exposure).

Once the halide concentration is known, the analysis subsystem 30 may use a same or different algorithm to calculate the total THMs present in analyte fluid. In exemplary embodiments, the algorithm includes multiplying the differential conductivity measurements by at least one compensation factor. The compensation factor(s) may include one or more efficiency corrections and/or polynomial coefficients, which may be derived from a regression analysis of samples with known quantities of THMs.

The algorithm may calculate the halide acid conductivity from the known halide conductivity and molar conductance of hydrogen ion and halide ions. For example, without limitation, for chlorides, the specific conductivity of the hydrogen ion is about 4.58 larger than the specific conductivity of the chloride ion. Further, the conductivity of halide acid may be calculated using molar conductance values.

From calibration data of conductivity of THMs vs concentration, the concentration of total THMs (TTHMs) may be calculated, which may be one of the one or more impurities. Unlike current THMs measurement methods such as gas chromatography and colorimetric-based analysers, this may provide ongoing measurements without the need for chemical reagents. The analysis subsystem 30 may calculate TTHMs concentrations through molar conductance values and electrochemical module 14 response, making it a unique and effective method for real-time monitoring of TTHMs contamination in purified water.

**Table 1: Molar conductance of hydrogen, chloride, bromide and iodide ions.**

| [0062] | Molar Conductance |
|---|---|
| H+ | 349.8 S-cm² mol⁻¹ |
| Cl- | 76.42 S-cm² mol⁻¹ |
| Br- | 78.40 S-cm² mol⁻¹ |
| I- | 76.8 S-cm² mol⁻¹ |

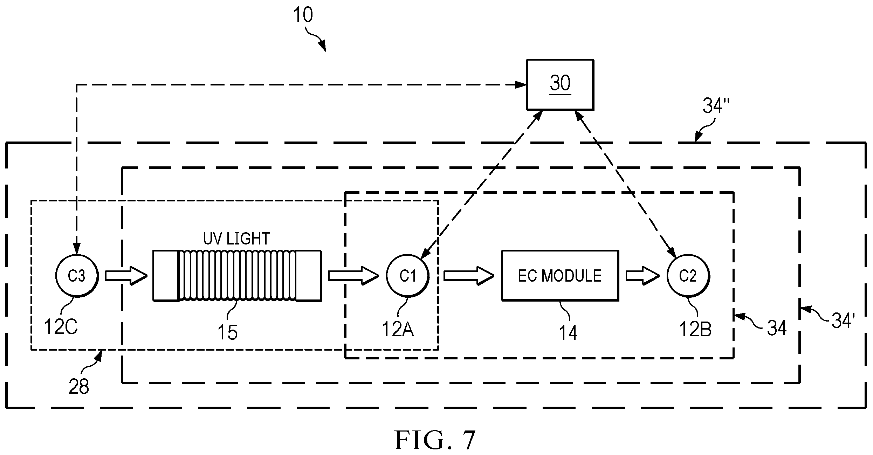

**FIGURE 7** illustrates another exemplary embodiment of the system 10, which may include the measurement subsystem 34 of figure 1, the measurement subsystem 34' of figure 6, and/or one or more components thereof.

A third conductivity sensor 12C may be provided upstream of the UV light source(s) 15 and/or other oxidation device(s). The third conductivity sensor 12C may be in electronic communication with the analysis subsystem 30 along with the first and second conductivity sensors 12A, 12B. At least the first, second, and third conductivity sensors 12A, 12B, 12C, the electrochemical module 14, and the UV light source(s) 15 and/or other oxidation device(s) may define and/or comprise at least part of another exemplary embodiment of the measurement subsystem 34". The analysis subsystem 30 may be part of, or separate from, the measurement subsystem 34". Stated another way, the measurement subsystem 34" may include the measurement subsystem 34' of figure 6 along with the third conductivity sensor 12C, and optionally any related components (e.g., power supplies, controllers, fluid passageway(s), combinations thereof, or the like) for the same.

The analysis subsystem 30 may be configured to receive conductivity data from the conductivity sensors 12A-12C, where the measurements from the third conductivity sensor 12C is sometimes referred to herein as "C3". A difference between conductivity measurements from the first and third conductivity sensors 12A, 12C (e.g., C1 - C3), respectively, may provide an estimated TOC measurement. In this way, the first and third conductivity sensors 12A, 12C and UV light source(s) 15 and/or other oxidation device(s), may provide a TOC analysis subsystem 28.

The analysis subsystem 30 may be configured to analyze the difference between conductivity measurements from the first and third conductivity sensors 12A, 12C, respectively (e.g., C1-C3; the "TOC difference") as well as the difference between conductivity measurements from the first and second conductivity sensors 12A, 12B, respectively (e.g., C2-C1; the "THM difference"). In this way, the analysis subsystem 30 may be configured to determine an estimated, interference compensated TOC measurement, which may be one of the one or more impurities. Alternatively, or additionally, the analysis subsystem may be configured to determine and/or provide the estimated TOC concentration and/or the THM concentration.

In exemplary embodiments, the algorithm includes multiplying the differential conductivity measurements (e.g., TOC difference and THM difference, separately) by at least one respective compensation factor. The compensation factor(s) may include one or more efficiency corrections and/or polynomial coefficients, which may be derived from a regression analysis of samples with known quantities of TOCs and/or THMs. In exemplary embodiments, without limitation, equation 6 is utilized to measure THMs along with a three-dimensional error analysis mesh, such as to compensate for temperature and/or other factors, to determine estimated TOC concentration based on the TOC difference.

By placing the electrochemical module 14 downstream of the first conductivity sensor 12A in the TOC analysis subsystem 28 and the second conductivity sensor 12B downstream of the electrochemical module 14, the halide ions from halide acid electrochemically chemisorbed on the silver electrode surface in the form of silver halide under the application of potential which results in loss of halide conductivity in the analyte fluid. Then, conductivity of the analyte fluid, free from halides, can be measured at the second conductivity sensor 12B. Difference in conductivities of C1 and C2 may be used by the analysis subsystem 30 to calculate the halide concentration in the water. Once the halide concentration is known, halide acid conductivity may be calculated as discussed with regard to at least figure 6 and conductivity of halide acid subtracted from the TOC measurements to correct the TOC recovery.

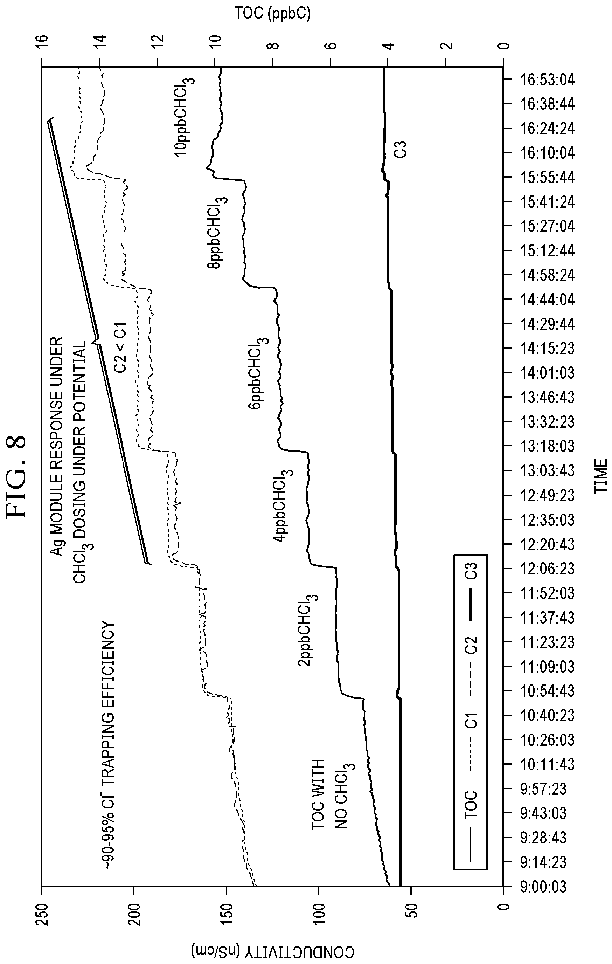

As illustrated in **FIGURE 8****,** by way of non-limiting example, different concentration of chloroform, CHCl3, dosing is given to the silver based electrochemical module under continuous potential. Stepwise increments in TOC and conductivity readings are seen due to chloroform dissociation to hydrochloric acid under UV exposure. Under potential, chloride ions are chemisorbed on the silver electrode surface resulting in loss of chloride conductivity in the analyte fluid. The conductivity measured at the second conductivity sensor 12B (C2) is smaller in magnitude (chloride conductivity free) as compared to conductivity at the first conductivity sensor 12A (C1) due to the loss of chloride conductivity in the water.

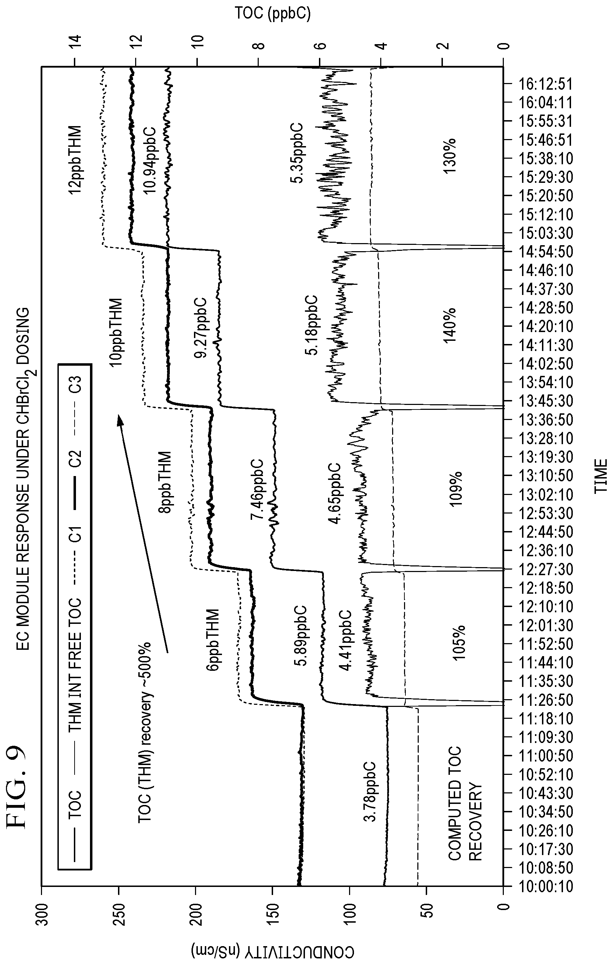

**FIGURE 9** is another exemplary dosing chart, including exemplary interference compensated TOC measurements.

The communicated data from the sensors 12 may comprise raw data, partially, and/or while processed data, such as with scaling, formatting, conversion, data compensation, noise removal, outlier removal, into one or more of various conductivity measures, combinations thereof, or the like.

The communicated data from the sensors 12 may be provided to the analysis subsystem 30 with an associated time or measurement and/or the analysis subsystem 30 may associated the received data with a time, such as based on a time of receipt. The analysis subsystem 30 may be configured to use time correlated data, preferably such that a same or substantially (e.g., within 51%) the same quantity of analyte is compared. In exemplary embodiments, without limitation, the analysis subsystem 30 may be configured to utilize data that is spaced apart temporally a same of different predetermined amount between the various sensors 12, such as based on estimated time for the same quantity of analyte to travel between the same. To provide a simplified example, conductivity measurements at a first time (T1) may be utilized from the first conductivity sensor 12A while conductivity measurements at a second time (T2), sometime thereafter, may be utilized from the second conductivity sensor 12B to account for the time it takes a sample to travel through the electrochemical module 14. This may provide time correlated conductivity measurements from the second conductivity sensor (C2) with the associated conductivity measurements from the first conductivity sensor (C1).

The flow of analyte may be ongoing (e.g., continuous). The analyte may be a sample portion of a larger system. The analysis subsystem 30 may be configured to analyze the received data and provide the related outputs on an ongoing (e.g., continuous) basis and/or in real-time or substantially real-time (e.g., accounting for normal delays in processing and/or transmission times, with no deliberate delay exceeding 10 minutes, by way of example).

The system 10 may operate without the need for batching, by way of example.

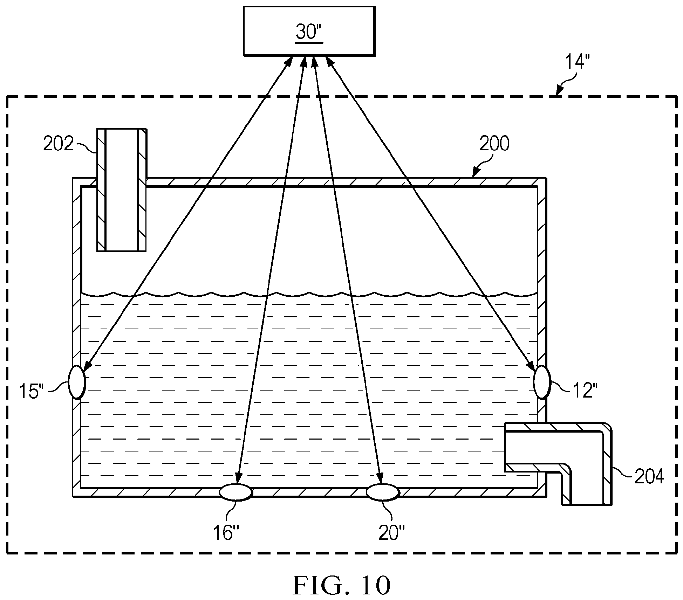

In other exemplary embodiments, without limitation, batching may be utilized. As illustrated with particular regard to **FIGURE 10****,** by way of non-limiting example, a batch processing container 200 may be provided and/or utilized. The container 200 may include an electrochemical module 14". The electrochemical module 14" may include some or all of the components and/or be operated using some or all of the methods shown and/or described herein, such as with regard to the electrochemical module 14 and/or 14'. The electrochemical module 14" may include at least one anode 16", preferably comprising sliver by way of non-limiting example, and at least one cathode 20", preferably comprising at least one of stainless steel, platinum, titanium, silver, gold, graphite, and carbon by way of non-limiting example. Various number and/or arrangement of the anode(s) 16" and cathode(s) 20" may be utilized. The inert material 18, 18' may not be required. The anode(s) 16" and/or cathode(s) 20" may be provided at one or more walls of the container 200, within the container 200, combinations thereof, or the like.

At least one conductivity sensor 12" may be provided. Various number and/or arrangement of the conductivity sensor(s) 12" may be utilized. The conductivity sensor(s) 12" may be provided at one or more walls of the container 200, within the container 200, combinations thereof, or the like.

One or more UV light source(s) 15" may be provided at the container 200. Various number and/or arrangement of the UV light source(s) 15" may be utilized. The UV light source(s) 15" may be provided at one or more walls of the container 200, within the container 200, combinations thereof, or the like.

A batch of analyte fluid may be periodically moved into the container 200, such as by way of an entrance 202 and one or more fluid control devices (e.g., pumps, valves, or the like). Multiple entrance(s) 202 may be provided, and the location of such entrance(s) 202 need not necessarily be as illustrated. Measurements may be made of the batch of analyte fluid by the analysis subsystem 30" of conductivity, such as by way of the conductivity sensor(s) 12", such as before operating the UV light source(s) 15" and/or activating the electrochemical module 14". The batch of analyte fluid may be exposed for a period of time (e.g., to UV light from the UV light source(s) 15" and/or the activated electromedical module 14") to effectuate dosing. The batch of analyte fluid may be measured, such as by way of the analysis subsystem 30", at various stages through the dosing (e.g., after UV light activation and/or after electromedical module 14" activation), such as to generate the same or similar data as shown and/or described herein. The batch of analyte fluid may be removed from the container 200, such as by way of an exit 204 and/or the same or different fluid control devices. Multiple exit(s) 204 may be provided, and the location of such exit(s) 204 need not necessarily be as illustrated.

The analysis subsystem 30" may be configured to exert operational control over the UV light source(s) 15", the electrochemical module 14", the conductivity sensor(s) 12", and/or the fluid control device(s).

The analysis subsystem 30 may be configured to electronically output the data in various forms, such as to one or more remote electronic devices (e.g., displays, smartphones, servers, computers, combinations thereof, or the like). The analysis subsystem 30 may be configured to store historical information, such as for generating summaries of the data over time in various forms.

The analysis subsystem 30 may be configured to analyze the data as part of determining the estimated concentrations of the one or more impurities, such as but not limited to, the estimated halide concentration, interference compensated TOC measurement, estimated TOC concentration, and/or estimated concentration of THMs and/or TTHMs, such as but not limited to, scaling, formatting, conversion, data compensation, noise removal, outlier removal, combinations thereof, or the like. Such analysis may include multiplying one or more of the measurements and/or differences by various coefficients or other factors. In exemplary embodiments, without limitation, the coefficients are derived from a regression analysis of conductivity-concentration curve of each of the one or more impurities.

The analysis subsystem 30 may be configured to generate alerts when the concentration levels of the one or more impurities are outside of one or more predetermined parameters. The parameter(s) may be impurity specific and/or variable based on other factors (e.g., temperature, flow rate, etc.). The parameter(s) may be user set and/or modified.

While certain components, such as the conductivity sensors 12A-12C are sometimes shown and/or described as being of a same or similar type and/or kind, they may be of different type and/or kind.

While certain items are sometimes described herein as a system 10 and/or as separate components, they may also be provided as a device and/or apparatus, such as but not limited to, within a single housing and/or structure or in multiple, connected and/or disconnected, housings and/or structures.

The system 10 may be provided as a standalone device, as part of a water purification system, as part of a water testing system, retrofitted to existing water systems or water testing systems, combinations thereof, or the like.

When anodes 16 and/or cathodes 20 of the electrochemical module 14 are exhausted, particularly when they comprise silver, they may be renewed (e.g., AgX (X=Cl, Br, I) to Ag) using various techniques, such as but not limited to, applying cathodic potential, use of reducing agents, light exposure (photodecomposition), temperature application, combination thereof, or the like.

The analysis subsystem 30 may be local to the measurement subsystem 34, 34', 34" or remote therefrom. The analysis subsystem 30 may comprise one or more non-transitory electronic storage devices and processors. The analysis subsystem 30 may comprise a server, computer, programmable logic circuit (PLC), closed appliance, combinations thereof, or the like.

Any embodiment of the present invention may include any of the features of the other embodiments of the present invention. The exemplary embodiments herein disclosed are not intended to be exhaustive or to unnecessarily limit the scope of the invention. The exemplary embodiments were chosen and described in order to explain the principles of the present invention so that others skilled in the art may practice the invention. Having shown and described exemplary embodiments of the present invention, those skilled in the art will realize that many variations and modifications may be made to the described invention. Many of those variations and modifications will provide the same result and fall within the spirit of the claimed invention.

Certain operations described herein may be performed by one or more electronic devices. Each electronic device may comprise one or more processors, electronic storage devices, executable software instructions, combinations thereof, and the like configured to perform the operations described herein. The features and/or functionality shown and/or described herein may be accomplished by way of stored, executable software instructions, such as in the form of applications, programs, routines, combinations thereof, or the like. The electronic devices may be general purpose computers or specialized computing devices. The electronic devices may comprise personal computers, smartphones, tablets, databases, servers, or the like. The electronic connections and transmissions described herein may be accomplished by one or more wired or wireless connectivity components (e.g., routers, modems, ethernet cables, fiber optic cable, telephone cables, signal repeaters, and the like) and/or networks (e.g., internets, intranets, cellular networks, the world wide web, local area networks, and the like). The computerized hardware, software, components, systems, steps, methods, and/or processes described herein may serve to improve the speed of the computerized hardware, software, systems, steps, methods, and/or processes described herein. The electronic devices, including but not necessarily limited to the electronic storage devices, databases, controllers, or the like, may comprise and/or be configured to hold, solely non-transitory signals.

## Claims

1. A system (10) for measuring estimated concentrations of one or more impurities in water, said system comprising:
a measurement subsystem (34) for a flow of analyte, said measurement subsystem (34) comprising:
a first conductivity sensor (12A);
an electrochemical module (14) positioned downstream of the first conductivity sensor (12A); and
a second conductivity sensor (12B) positioned downstream of the electrochemical module (14);
an analysis subsystem (30) in electronic communication with the measurement subsystem (34), said analysis subsystem (30) comprising one or more non-transitory electronic storage devices comprising software instructions, which when executed,
configure one or more processors to:
receive measurement data from the measurement subsystem (34) for the flow of the analyte, said measurement data comprising conductivity measurements from the first conductivity sensor (12A) and the second conductivity sensor (12B);
determine a difference between a conductivity measurement from the second conductivity sensor (12B) and an associated conductivity measurement from the first conductivity sensor (12A); and
analyze the difference to determine an estimated concentration of each of the one or more of the impurities.

2. The system of claim 1 wherein:
the one or more non-transitory electronic storage devices of the analysis subsystem comprise additional software instructions, which when executed, configure the one or more processors to:
time correlate the conductivity measurements from the second conductivity sensor (12B) with the associated conductivity measurements from the first conductivity sensor (12A).

3. The system of claim 1 or 2 wherein:
the analysis subsystem (30) is configured to output the estimated concentration of halide.

4. The system of one of the preceding claims wherein:
the electrochemical module (14) comprises:
one or more anodes (16A-16F), each comprising silver;
one or more cathodes (20A-20F), arranged in an alternating fashion with the one or more anodes (16A-16F); and
inert material (18A-18K) interposed between each of the one or more anodes (16A-16F) and adjacent one of the one or more cathodes (18A-18K),
preferably each of the one or more of cathodes (20A-20F) comprise one or more of: stainless steel, platinum, titanium, silver, gold, graphite, and carbon, or
preferably the inert material (18A-18K) comprises at least one of: polyvinylidene fluoride, polyether ether ketone, perfluoroalkoxy alkane, and polypropylene, or
preferably each of the one or more anodes (16A-16F) each comprise a plurality of pores for the flow of the analyte to travel through; and each of the one or more cathodes (20A-20F) comprise a plurality of pores for the flow of the analyte to travel through, or
preferably each of the one or more anodes (16A-16F) and each of the one or more cathodes (20A-20F) are provided in the form of sheets which are stacked in an alternating fashion with the inert material (18A-18K) interposed therebetween to allow the flow of the analyte to travel therebetween.

5. The system of one of the preceding claims wherein:
the measurement subsystem (34) comprises one or more oxidation devices; and
said one or more oxidation devices is positioned upstream of the first conductivity sensor (12A).

6. The system of one of the preceding claims wherein:
the one or more oxidation devices comprise an ultraviolet light source (15); and
the analysis subsystem (30) is configured to output the estimated concentration of trihalomethane.

7. The system of claim 6 wherein:
the ultraviolet light source (15), when activated, is configured to generate photons shorter than 200 nm wavelength.

8. The system of one of the preceding claims wherein:
the measurement subsystem comprises a third conductivity sensor (12C) located upstream of the one or more oxidation devices;
the measurement data comprises conductivity measurements from the third conductivity sensor (12C); and
the one or more non-transitory electronic storage devices of the analysis subsystem (30) comprise additional software instructions, which when executed, configure the one or more processors to:
determine a difference between the conductivity measurement from the first conductivity sensor (12A) and an associated conductivity measurement from the third conductivity sensor (12C) ("second difference") to determine an estimated concentration of total organic carbon; and
analyze the second difference with the difference to determine an interference compensated estimated concentration of total organic carbon.

9. The system of one of the preceding claims wherein:
the one or more non-transitory electronic storage devices of the analysis subsystem (30) comprise additional software instructions, which when executed, configure the one or more processors to:
receive the measurement data, determine the difference, and analyze the difference in substantially real time on an ongoing basis such that the estimated concentration of each of the one or more of the impurities is determined over time,
preferably the one or more non-transitory electronic storage devices of the analysis subsystem comprise additional software instructions, which when executed, configure the one or more processors to:
transmit the estimated concentration of each of the one or more of the impurities to at least one remote electronic device for display.

10. The system of one of the preceding claims wherein:
the components of the measurement subsystem (34) are in direct fluid connection with one another; and
the analysis subsystem (30) is remote from the measurement subsystem.

11. The system of one of the preceding claims wherein:
the one or more non-transitory electronic storage devices of the analysis subsystem (30) comprise additional software instructions, which when executed, configure the one or more processors to:
analyze the difference to determine the estimated concentration of each of the one or more of the impurities at least in part by multiplying the difference by one or more coefficients.

12. The system of claim 11 wherein:
the one or more coefficients comprise a plurality of coefficients, at least one of which is derived by the regression analysis of the conductivity-concentration curve of a respective one of the one or more impurities.

13. The system of claim 12 wherein:
the measurement subsystem comprises (34) an ultraviolet light source (15) positioned upstream of the first conductivity sensor (12A) and a third conductivity sensor (12C) located upstream of the ultraviolet light source (15);
the measurement data comprises conductivity measurements from the third conductivity sensor (12C); and
the one or more non-transitory electronic storage devices of the analysis subsystem (30) comprise additional software instructions, which when executed, configure the one or more processors to:
determine a difference between the conductivity measurement from the first conductivity sensor (12A) and an associated conductivity measurement from the third conductivity sensor (12C) ("second difference") to determine an estimated concentration of total organic carbon; and
analyze the second difference with the difference to determine an interference compensated estimated concentration of total organic carbon, wherein interference compensated estimated concentration of total organic carbon includes compensation for the one or more impurities which comprise trihalomethane and halide.

14. A method for measuring estimated concentrations of one or more impurities in water, said method comprising:
providing a flow of analyte through a measurement subsystem (34) comprising:
a first conductivity sensor (12A);
an electrochemical module (14) positioned downstream of the first conductivity sensor (12A); and
a second conductivity sensor (12B) positioned downstream of the electrochemical module (14);
receiving, at an analysis subsystem (30) in electronic communication with the measurement subsystem (34), measurement data for the flow of the analyte, said measurement data comprising conductivity measurements from the first conductivity sensor (12A) and the second conductivity sensor (12B);
determining, at the analysis subsystem (30), a difference between a conductivity measurement from the second conductivity sensor (12B) and an associated conductivity measurement from the first conductivity sensor (12A); and
analyzing, at the analysis subsystem (30), the difference to determine an estimated concentration of each of the one or more of the impurities.

15. A system (10) for measuring estimated concentrations of one or more impurities in water, said system comprising:
a measurement subsystem (34") for a flow of analyte, said measurement subsystem comprising:
an ultraviolet (UV) light source (15), which when activated, generates photons shorter than 200 nm wavelength;
a first conductivity sensor (12A) positioned downstream of the UV light source;
an electrochemical module (14) positioned downstream of the first conductivity sensor (12A) and comprising a plurality of anodes, each comprising silver, a plurality of cathodes, each comprising stainless steel and provided in an alternating, stacked fashion with the anodes, and inert material comprising at least one of polyvinylidene fluoride, polyether ether ketone, perfluoroalkoxy alkane, and polypropylene interposed between each anode-cathode pair;
a second conductivity sensor (12B) positioned downstream of the electrochemical module (14); and
a third conductivity sensor (12C) located upstream of the UV light source (15);
an analysis subsystem (30) in electronic communication with the measurement subsystem (34"), said analysis subsystem (30) comprising one or more non-transitory electronic storage devices comprising software instructions, which when executed, configure one or more processors to, in substantially real time on an ongoing basis:
receive measurement data from the measurement subsystem for the flow of the analyte, said measurement data comprising conductivity measurements from the first conductivity sensor (12A), the second conductivity sensor (12B), and the third conductivity sensor (12C);
time correlate the conductivity measurements;
determine a difference between a conductivity measurement from the second conductivity sensor (12B) and an associated conductivity measurement from the first conductivity sensor (12A) ("first difference");
determine a difference between the conductivity measurement from the first conductivity sensor (12A) and an associated conductivity measurement from the third conductivity sensor (12C) ("second difference") to determine an estimated concentration of total organic carbon;
analyze the second difference with the difference to determine an interference compensated estimated concentration of total organic carbon, wherein interference compensated estimated concentration of total organic carbon includes compensation for the one or more impurities which comprise trihalomethane and halide, and where the analysis includes multiplying the difference by one or more coefficients derived by the regression analysis of the conductivity-concentration curve of a respective one of the one or more impurities; and
transmit the estimated concentration of each of the one or more of the impurities to at least one remote electronic device for display.
